# EUROPEAN PATENT SPECIFICATION

(11) **EP 0 691 980 B1**
(45) Date of publication and mention of the grant of the patent: **04.06.1997**
(21) Application number: 94913923.2
(22) Date of filing: 21.03.1994
(51) Int. Cl.: C07H 21/00

(54) **7-DEAZAPURINE MODIFIED OLIGONUCLEOTIDES**
7-DEAZAPURIN MODIFIZIERTE OLIGONUKLEOTIDE
OLIGONUCLEOTIDES MODIFIES CONTANT DES NUCLEOSIDES 7-DEAZAPURINES

(30) Priority: 30.03.1993 US 40158
(43) Date of publication of application: 17.01.1996
(73) Proprietor: SANOFI, 75374 Paris Cédex 08 (FR)
(72) Inventor: COOK, Phillip, D., Carlsbad, CA 92009 (US); DELECKI, Daniel, J., Radnor, PA 19087 (US)
(74) Representative: Le Guen, Gérard
(86) International application number: US9402996
(87) International publication number: WO9422892

(56) References cited:
- EP-A- 0 286 028
- NUCLEIC ACIDS RESEARCH. vol. 12, no. 23 , 11 December 1984 , ARLINGTON, VIRGINIA US pages 8939 - 8949 A.ONO ET AL. 'Synthesis of Deoxyoligonucleotides Containing 7-deazaadenine: Recognition and Cleavage by Restriction Endonuclease Bgl II and Sau 3AI (Nucleosides and Nucleotides Part 55).'
- BIOCHEMISTRY. vol. 26, no. 25 , 15 December 1987 , EASTON, PA US pages 8221 - 8227 H-N. WU ET AL. 'Role of a Bulged A Residue in a Specific RNA-Protein Interaction.'
- BIOCHEMISTRY. vol. 24, no. 26 , 17 December 1985 , EASTON, PA US pages 7556 - 7561 F.SEELA ET AL. 'Oligomers with ALternating Thymidine and 2'-Deoxytubercidin: Duplex Stabilization by a 7-Deazapurine Base.' cited in the application
- BIOCHEMISTRY. vol. 28, no. 15 , 25 July 1989 , EASTON, PA US pages 6193 - 6198 F.SEELA ET AL. 'Bending of Oligonucleotides Containing an Isosteric Nucleobase: 7-Deaza-2'-deoxyguanosine Replacing dA within d(A)6 Tracts.'
- BIOCHEMISTRY. vol. 21, no. 18 , 31 August 1982 , EASTON, PA US pages 4338 - 4343 F.SEELA ET AL. 'Poly(7-deazaguanylic Acid), the Homopolynucleotide of the Parent Nucleoside of Queuosine.'
- TETRAHEDRON, (INCL. TETRAHEDRON REPORTS) vol. 41, no. 22 , 1985 , OXFORD GB pages 5387 - 5392 F.SEELA ET AL. '2'-Desoxytubercidin: Synthese des O-3'-Phosphoramidites und Kondensation zu 2'-Desoxytubercidylyl(3'- 5')-2'-Desoxytubercidin.' cited in the application
- BIOCHEMISTRY. vol. 29, no. 42 , 23 October 1990 , EASTON, PA US pages 9891 - 9901 P.C.NEWMAN ET AL. 'Incorporation of a Complete Set of Deoxyadenosine and Thymidine Analogues Suitable for the STudy of Protein Nucleic Acid Interactions into Oligodeoxynucleotides. Application to the EcoRV Restriction Endonuclease and Modification Methylase.'
- BIOCHEMISTRY. vol. 29, no. 42 , 23 October 1990 , EASTON, PA US pages 9902 - 9910 P.C.NEWMAN ET AL. 'Interaction of the EcoRV Restriction Endonuclease with the Deoxyadenosine and Thymidine Bases in its Recognition Hexamer d(GATATC).'
- BIOCHEMISTRY. vol. 26, no. 8 , 21 April 1987 , EASTON, PA US pages 2232 - 2238 F.SEELA ET AL. 'Palindromic Octa- and Dodecanucleotides Containing 2'-Deoxytubercidin: Synthesis, Hairpipn Formation, and Recognition by the Endodeoxyribonuclease EcoRI.' cited in the application
- NUCLEIC ACIDS RESEARCH. vol. 16, no. 24 , 23 December 1988 , ARLINGTON, VIRGINIA US pages 11781 - 11793 A.FLIESS ET AL. 'Analysis of the Recofnition Mechanism Involved in the EcoRV Catalyzed Cleavage of DNA using Modified Oligodeoxynucleotides.'
- NUCLEIC ACIDS RESEARCH. vol. 17, no. 3 , 11 February 1989 , ARLINGTON, VIRGINIA US pages 901 - 910 F.SEELA ET AL. 'Alternating d(G-C)3 and d(C-G)3 Hexanucleotides containing 7-deaza-2'-deoxyguanosine ir 8-aza-7-deaza-2'-deoxyguanosine in Place of dG.' cited in the application
- NUCLEIC ACIDS RESEARCH. vol. 14, no. 5 , 11 March 1986 , ARLINGTON, VIRGINIA US pages 2319 - 2332 F.SEELA ET AL. 'Palindromic Oligonucleotides Containing 7-deaza-2'-deoxyguanosine: Solid-Phase Synthesis of d((p)GG*AATTCC) Octamers and Recognition by the Endodeoxyribonuclease EcoRI.' cited in the application
- NUCLEIC ACIDS RESEARCH. vol. 13, no. 3 , 11 February 1985 , ARLINGTON, VIRGINIA US pages 911 - 925 F.SEELA ET AL. 'Solid-Phase Synthesis of the Self-Complementary Hexamer d(c7GpCpc7GpCpc7GpC) via the O-3'-Phosphoramidite of 7-deaza-2'-deoxyguanosine.'
- Biochemistry 28, pp6198-6207.1989

## Description

This invention relates to modified oligonucleotide sequences containing 7-deazapurine nucleosides, to a method of inhibiting nuclease degradation of oligonucleotides incorporating the same, to a method of inhibiting gene expression in a cellular system and to compositions useful for inhibiting gene expression containing the modified oligonucleotides.

### Information Disclosure Statement

Seela and Kehne, Biochem., 26, 2232-2238 (1987) disclose 7-deazadeoxyadenosine (9-β-2'-deoxyribofuranosyl-7-deazaadenine) and the incorporation of from one to two such nucleosides into octa and dodecanucleotides having the palindromic EcoRI endonuclease DNA recognition sequence d(GAATTC). The oligonucleotides were prepared for study of their stability to cleavage by EcoRI.

Seela and Driller, Nucl. Acid. Res., 17(3), 901-910 (1989) describe the preparation of hexanucleotide sequences containing d(GC)₃ and d(CG)₃ nucleotide units and such hexamers containing 7-deazaguanosine (c⁷G_{d}) and 7-deaza-8-azaguanosine (c⁷z⁸G_{d}) nucleoside units. The self-complementary hexamers so-prepared form duplexes which were prepared for the purpose of studying the stability of the duplexes and the thermodynamic parameters of helix-coil transition for each of the G-C/C-G base pairs.

Tran-Thi et al., Angew. Chem. Int. Ed. Engl., 21(5), 367-368, (1982) disclose the preparation of 7-deazaguanosine and the preparation therefrom of cyclic guanosine monophosphate.

Seela and Kehne, Biochem., 24(26), 7556-7561 (1985) describe the synthesis of self-complementary hexamers and dodecamers employing solid phase techniques and an appropriately protected phosphoramidite for study of the base pairing and base stacking properties as reflected by their melting curves and their behavior toward snake venom phosphodiesterase and single strand specific nuclease SI.

Seela and Driller, Nucl. Acid. Res., 13(3), 911-926 (1985) describe the synthesis of a 3'-phosphoramidite of 7-deaza-2'-deoxyguanosine and the synthesis of the self-complementary hexamer of d(CG), where the guanosine moieties were replaced by 7-deaza-2'-deoxyguanosine, and study of the properties of the resulting duplex.

Winkeler and Seela, J. Org. Chem., 48, 3119-3122 (1983) report the total synthesis of 7-deaza-2'-deoxyguanosine and its incorporation into oligo and polynucleotides for the purpose of providing information on base pairing and enzyme recognition is in progress.

Seela and Kehne, Tetrahedron, 41(22), 5387-5392 (1985) disclose the preparation of 2'-deoxytubercidylyl(3'→5')-2'-deoxytubercidin, i.e. 7-deaza-2'-deoxyadenosinyl(3'→5')-7-deaza-2'-deoxyadenosine, for study of its hypochromicity and its stability to nuclease cleavage, by condensation of 3'-O-[(N,N-diisopropylamino)methoxyphosphanyl]-5'-O-(4,4'-dimethoxytrityl)-7-deaza-6-benzoyl-2'-deoxyadenosine with 3'-O-(t-butylmethylsilyl)-7-deaza-2'-deoxyadenosine and removal of the O-methyl, O-dimethoxytrityl and N-benzoyl protecting groups.

Winkeler and Seela, Liebigs Ann. Chem., 708-721 (1984) disclose 7-deaza-7-methyl-2'-deoxyguanosine and its synthesis from 2,4-diamino-6-hydroxypyrimidine.

Seela and Kehne, Liebigs Ann. Chem., 876-884 (1983) disclose the preparation of 7-deaza-2'-deoxyadenosine.

Seela and Driller, Nucl. Acid. Res., 14, 2319-2332 (1986) disclose the preparation, by solid phase synthesis via the nucleoside phosphoramidites, of octadecanucleotides containing the EcoRI endonuclease recognition site and 7-deaza-2'-deoxyguanine and kinetic studies on their cleavage by EcoRI.

Seela, Tran-Thi and Franzen, Biochem., 21, 4338-4343 (1982) disclose the preparation of polymers of 7-deazaguanosine for study of their hypochromicity, melting profiles and circular dichroism spectra.

EPO Application 286,028, published October 12, 1988, discloses 7-deazapurine nucleosides of the formula: 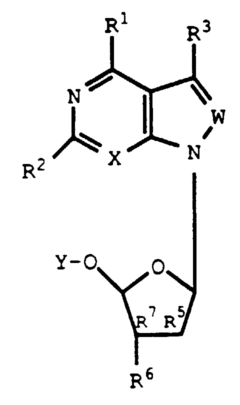 where:
X is N or a =CH group;
W is N or a =CR⁴ group;
R¹, R², R³ and R⁴ are the same or different hydrogen, halogen, lower-alkyl, hydroxy, mercapto, lower-alkylthio, lower-alkoxy, arylalkyl, arylalkoxy, aryloxy or a mono or di-substituted amino group;
R⁵ is hydrogen or hydroxy;
R⁶ and R⁷ are hydrogen or one or both can be halogen, cyano, azido or a mono or di-substituted amino group, and wherein one of R⁶ and R⁷ can be hydroxy when X is a =CH group and furthermore R⁵ and R⁷ together can be a second bond between the C₂' and C₃' positions and Y is hydrogen or a mono, di or triphosphate.

The compounds are stated to be useful in nucleic acid sequencing, and as antiviral agents

### BACKGROUND OF THE INVENTION

An antisense compound is a compound that binds to or hybridizes with a nucleotide sequence in a RNA or DNA to inhibit the function or synthesis of tne nucleic acid. Because of their ability to hybridize with both RNA and DNA, antisense compounds can interfere with gene expression at the level of transcription, RNA processing or translation.

Antisense molecules can be designed and synthesized to prevent the transcription of specific genes to mRNA by hybridizing with genomic DNA and directly or indirectly inhibiting the action of RNA polymerase. A theoretical advantage of targeting DNA is that only small amounts of antisense compounds may be needed to achieve a therapeutic effect. Alternatively, antisense compounds can be designed and synthesized to hybridize with RNA to inhibit post-transcriptional modification (RNA processing) or protein synthesis (translation) mechanisms or to affect mRNA stability. Exemplary target RNAs are messenger RNA (mRNA), transfer RNA (tRNA), ribosomal RNA (rRNA) heterogenous nuclear RNA (hnRNA) and the like. Examples of processing and translation mechanisms include splicing of pre-mRNA to remove introns, capping of the 5' terminus of mRNA, transport to the cytoplasm, hybridization arrest and ribonuclease H mediated mRNA hydrolysis.

At the present time, however, the development of practical scientific and therapeutic applications of antisense technologies is hampered by a number of technical problems. Synthetic antisense molecules are susceptible to rapid degradation by nucleases that exist in target cells. The oligonucleotide sequences of antisense DNA or RNA, for example, are destroyed by exonucleases acting at either the 5' or 3' terminus of the nucleic acid. In addition, endonucleases can cleave the DNA or RNA at internal phosphodiester linkages between individual nucleotides. As a result of such cleavage, the effective half-life of administered antisense compounds is very short, necessitating the use of large, frequently administered, dosages.

Another problem is the extremely high cost of producing antisense DNA or RNA using available semiautomatic DNA synthesizers.

A further problem relates to the delivery of antisense agents to desired targets within the body and cell. Antisense agents targeted to genomic DNA must gain access to the nucleus (i.e. the agents must permeate the plasma and the nuclear membrane). The need for increased membrane_permeability (increased hydrophobicity) must be balanced, however, against the need for aqueous solubility (increased hydrophilicity) in body fluid compartments such as the plasma and cell cytosol.

A still further problem relates to the stability of antisense agents whether free within the body or hybridized to target nucleic acids. Oligonucleotide sequences such as antisense DNA are susceptible to steric reconfiguration around chiral phosphorus centers.

Gene targeting via antisense agents is the predicted next step in human therapeutics [Armstrong, Business Week March 5, 1990, page 88]. The successful application of antisense technology to the treatment of disease, however, requires finding solutions to the problems set forth above.

One approach to preparing antisense compounds that are stable, nuclease resistant, inexpensive to produce and which can be delivered to and hybridize with nucleic acid targets throughout the body is to synthesize oligonucleotide sequences having incorporated therein modified adenine or guanine purine bases which are capable of hybridizing with their complementary respective thymine or cytosine bases but which are less susceptible to attack by exo- or endonucleases and which thus stabilize the oligonucleotide sequences to enzymatic degradation This invention is directed to such an approach.

### SUMMARY OF THE INVENTION

In a product aspect the invention relates to oligonucleotides incorporating a sequence of the normal DNA bases, adenine (A), thymine (T), guanine (G) and cytosine (C) in the required sequences for hybridization with a given DNA or RNA base sequence and in which one or more of the normal bases are replaced by a modified 7-deaza-7-lower-alkyladenine or 7-deaza-7-lower-alkylguanine.

In a method aspect, the invention relates to a method of inhibiting nuclease degradation of oligonucleotides comprising incorporating one or more modified 7-deaza-7-lower-alkyladenine or 7-deaza-7-lower-alkylguanine nucleosides into a normal DNA base sequence.

In a further method aspect, the invention relates to a method of inhibiting gene expression in a cellular system comprising introducing into a cellular system a composition containing an oligonucleotide which incorporates one or more modified 7-deaza-7-lower-alkyladenine or 7-deaza-7-lower-alkylguanine nucleosides.

In a composition aspect, the invention relates to compositions for inhibiting gene expression comprising an oligonucleotide which incorporates one or more modified 7-deaza-7-lower-alkyladenine or 7-deaza-7-lower-alkylguanine nucleosides in a pharmaceutically acceptable carrier.

### DESCRIPTION OF THE PREFERRED EMBODIMENTS

More specifically this invention relates to oligonucleotides incorporating a sequence of nucleotides of the normal DNA bases, i.e. adenine, thymine, guanine and cytosine, in the required sequence for hybridization with a given DNA or RNA base sequence and in which one or more of the normal bases are replaced by a 7-deazaadenine-β-D-ribofuranosyl or β-D-2'-deoxyribofuranosyl nucleoside of the formula: 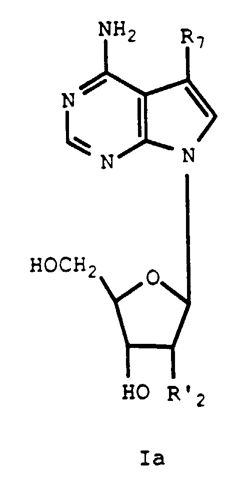 or a 7-deazaguanine-β-D-ribofuranosyl- or β-D-2'-deoxyribofuranosylnucleoside of the formula: 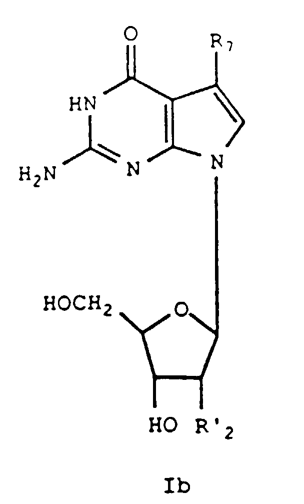 where
R'₂ is hydrogen or hydroxy; and
R₇ is lower-alkyl containing one to four carbon atoms with the provisos that the oligonucleotides incorporate (1) no EcoRI endonuclease recognition sites, (2) no repeating GC or CG sequences, and (3) no repeating AT or TA sequences, said 7-deaza-7-lower-alkyladenine nucleosides of formula Ia and said 7-deaza-7-lower-alkyl-guanine nucleosides of formula Ib being :

- either incorporated within the three nucleotide units at both the 3'- and the 5'- ends of the oligonucleotide sequence;
- or incorporated internally in the oligonucleotide sequence.

Preferred oligonucleotides within the ambit of the invention are those which incorporate from one to four of the 7-deaza-7-lower-alkyladenine or 7-deaza-7-lower-alkylguanine nucleosides of formulas Ia or Ib in the polymer.

The oligonucleotides of the invention are particularly stable to exonuclease degradation.

Particularly preferred oligomers are those whicn incorporate nucleotides derived from the nucleosides of formula Ia wherein:
R'₂ is hydrogen and R₇ is lower-alkyl, i.e. 7-deaza-2'-deoxy-7-methyladenosine (9-β-D-2'-deoxyribofuranosyl-7-deaza-7-methyladenine), hereinafter identified as nucleotide X; and
R'₂ is hydroxy and R₇ is lower-alkyl, i.e. 7-deaza-7-methyladenosine (9-β-D-ribofuranosyl-7-deaza-7-methyladenine), hereinafter identified as nucleotide X';
and nucleosides of formula Ib wherein:
R'₂ is hydrogen and R₇ is lower-alkyl, i.e. 7-deaza-2'deoxy-7-methylguanosine (9-β-D-2'-deoxyribofuranosyl-7-deaza-7-methylguanine), hereinafter identified as nucleotide Z; and
R₂ is hydroxy and R₇ is lower-alkyl, i.e. 7-deaza-7-methylguanosine (9-β-D-ribofuranosyl-7-deaza-7-methylguanine), hereinafter identified as nucleotide Z'.

As used herein the term lower-alkyl means a saturated, aliphatic, straight or branched chain hydrocarbon radical containing from one to four carbon atoms and thus includes methyl, ethyl, propyl, isopropyl and butyl.

The oligomers useful in the practice of the invention comprise sequences of from about 6 to 200 bases, preferably from about 12 to about 24 bases, and most preferably 15 bases in which one or more of the nucleosides are replaced by the modified 7-deaza-7-lower-alkyladenine and 7-deaza-7-lower-alkylguanine nucleosides of formulas Ia or Ib.

The oligonucleotides of the invention are prepared by solid phase synthesis according to well known procedures [Sinha et al., Nucl. Acid Res., 12, 4539-4557 (1984)] from protected 9-[3'-O-[(N,N-diisopropylamino) (R'₃-oxy)phosphanyl]-5'-O-(4,4'-dimethoxytrityl)purine nucleosides of the formula: 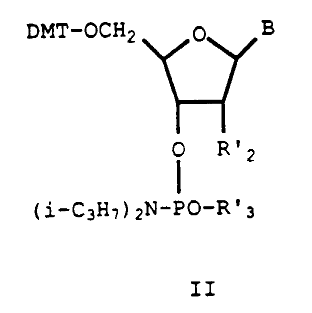 where
B represents the bases adenine (A), thymine (T), cytosine (C), guanine (G) or the modified 7-deaza-7-lower-alkyladenine or 7-deaza-7-lower-alkylguanine bases corresponding to the bases in the nucleosides of formulas Ia and Ib;
DMT represents the dimethoxytrityl group (i.e., the 4,4'-dimethoxytriphenylmethyl group); and
R'₂ has the meanings given above and where the 2-amino or 6-amino group of the respective guanine/7-deazaguanine, adenine/7-deazaadenine moieties are protected with a protecting group such as a benzoyl or isobutyryl group.

Particularly preferred solid phase syntheses are those such as described by Matteucci and Caruthers, J. Am. Chem. Soc., 103, 3185-3191 (1981) and Gait, Oligonucleotide Synthesis:A Practical Approach, Ed. by M.J. Gait, 35-81, IRL Press, Washington, D.C. 1984.

The initial step in solid phase synthesis is attachment of a nucleoside to a solid support, preferably a controlled pore glass (CPG) support. The nucleoside is preferably attached to the CPG via a succinate linkage at the 3'-hydroxy position of the nucleoside. Other means of attaching nucleosides to solid supports are known and readily apparent to those skilled in the oligonucleotide synthesis art.

Following attachment of the first nucleoside to the solid support, chain elongation occurs via the sequential steps of removing the 5'-hydroxy protecting group, activating the 5'-hydroxy group in the presence of a phosphoramidite reagent, adding the desired nucleoside, capping the unreacted nucleoside and oxidizing the phosphorus linkage. The protecting group, preferably DMT, at the 5'-hydroxy position of the attached nucleoside is removed with acid, preferably trichloroacetic acid.

Activating reagents that can be used in accordance with this method are well known to those skilled in the art. Preferred activating reagents are tetrazole and activator gold (Beckman Instr. Inc., Palo Alto, CA).

The activation step occurs in the presence of the added nucleoside and a trityldiolcyanophosphine compound, which compound replaces the nucleoside phosphoramidite of conventional synthetic methods. Unreacted chains are terminated or capped with capping reagents such as acetic anhydride and N-methylimidazole.

The labile trivalent phosphorus linkage is oxidized, preferably with iodine, to the stable, pentavalent phosphodiester linkage of the oligonucleotide.

After the desired oligonucleotide chain assembly is complete, the phosphate protecting groups are removed, the chains are separated from the solid support and the base protecting groups are removed by conventional methods. (Gaits, supra at 67-70.)

The compounds of the present invention are useful in treating mammals with hereditary disorders or diseases associated with altered genetic expression mechanisms. Examples of such diseases are viral infections such as HIV, cytomegalovirus, herpes simplex, hepatitis B, papilloma virus and picornavirus; cancers of the lung, colon, cervix, breast and ovary; inflammatory diseases; and diseases of the immune system such as acquired immunodeficiency syndrome (AIDS), hematological neoplasma and hyperproliferative disorders. [Armstrong, supra at 89; Klausner, Biotechnology, 8, 303, 304 (1990).]

The protected purine nucleosides of formula II required for the preparation of the oligomers of the invention can be prepared by the methods described by Seela and Kehne (1987) supra; Seela and Kehne, Tetrahedron 41(22), 5387-5892 (1985); Seela and Driller (1989) supra; and Seela and Driller (1985) supra which involve benzoylation of unprotected amino groups in the unprotected nucleosides of formulas Ia and Ib, or the unprotected 7-deazapurine nucleosides of adenine, guanine or cytosine, formation of the 5'-dimethoxytrityl ether and converting the product to the 3-phosphoramidite.

The corresponding unprotected 7-deazapurine nucleosides of formula Ia and formula Ib can be prepared by the methods described by Seela and Kehne (1987) supra, Seela and Driller (1989) supra, Tran-Thi supra, Seela and Driller (1985) supra, Winkeler and Seela (1983) supra, Winkeler and Seela (1984) supra and Seela and Kehne (1983) supra. 7-Deaza-2'-deoxy-7-lower-alkyladenines (formula Ia, R₂=H) can be prepared by the following reaction scheme. 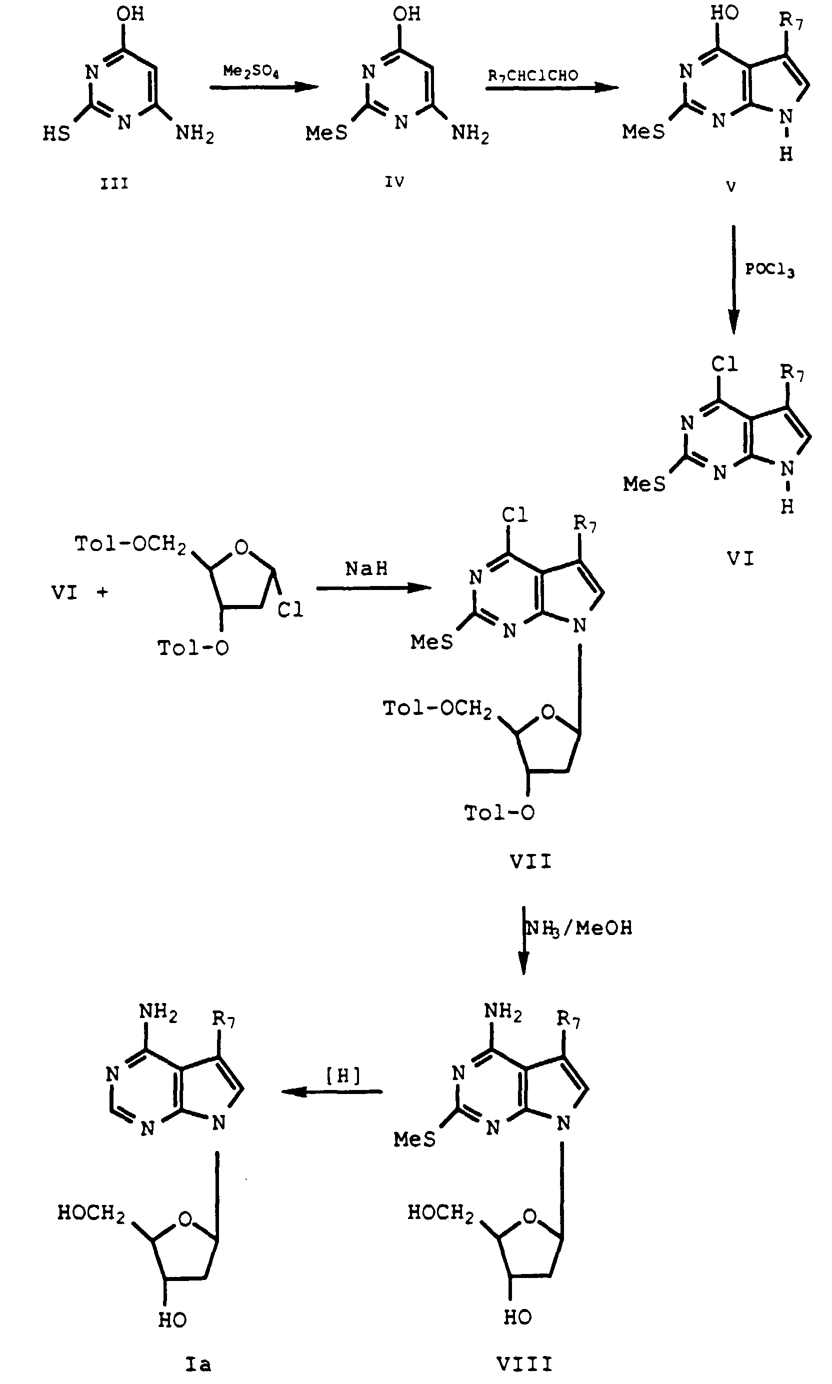 where R₇ is lower-alkyl, and Tol is the p-toluoyl group.

Thus 6-amino-2-thiouracil (III) is treated with an alkylating agent, for example dimethyl sulfate, methyl bromide or methyl iodide, in the presence of a base, for example an alkali metal carbonate, in an organic solvent such as dimethylformamide (hereinafter DMF) or acetone. Using the procedure described by Winkeler and Seela (1984) supra, the resulting 2-methylthio-4-amino-6-hydroxypyrimidine (IV) is converted to the compound of formula VII by reaction of the compound of formula IV with an appropriate 2-chloro-lower-alkanal in the presence of an alkali metal carbonate and a tetra-lower-alkyl ammonium halide, conversion of the resulting 4-hydroxy-2-methylthio-5-lower-alkyl-7H-pyrrolo[2,3-d]pyrimidine of formula V to the corresponding 4-chloro-2-methylthio-5-lower-alkyl-7H-pyrrolo[2,3-d]pyrimidine of formula VI which is reacted with 1-chloro-2-deoxy-3,5-di-O-(p-toluoyl)-α-D-erythropentofuranose in the presence of a base to give a 4-chloro-2-methylthio-5-lower-alkyl-7-[2'-deoxy-3',5'-di-O-(p-toluoyl)-β-D-erythro-pentofuranosyl]-7H-pyrrolo[2,3-d]pyrimidine of formula VII. Thereafter the compound of formula VII, in an appropriate organic solvent inert under the conditions of the reaction such as a lower-alkanol, is heated with ammonia in an autoclave to give the 4-amino-2-methylthio-5-lower-alkyl-7-[2'-deoxy-β-D-erythro-pentofuranosyl]-7H-pyrrolo[2,3-d]pyrimidine (alternatively named as 9-β-D-2'-deoxyribofuranosyl-2-methylthio-7-deaza-7-lower-alkyladenine) of formula VIII. Reductive dethiomethylation of the latter in an inert organic solvent, for example a lower alkanol, over Raney nickel affords the desired 9-β-D-2'-deoxyribofuranosyl-7-deaza-7-lower-alkyladenine of formula Ia wherein R₂' is hydrogen. The latter can then be converted to the protected 7-deaza-7-lower-alkyladenines of formula II as described before.

Likewise, 9-β-D-2'-deoxyribofuranosyl-7-deaza-7-lower-alkylguanine of formula Ib can be prepared by the following reaction scheme;

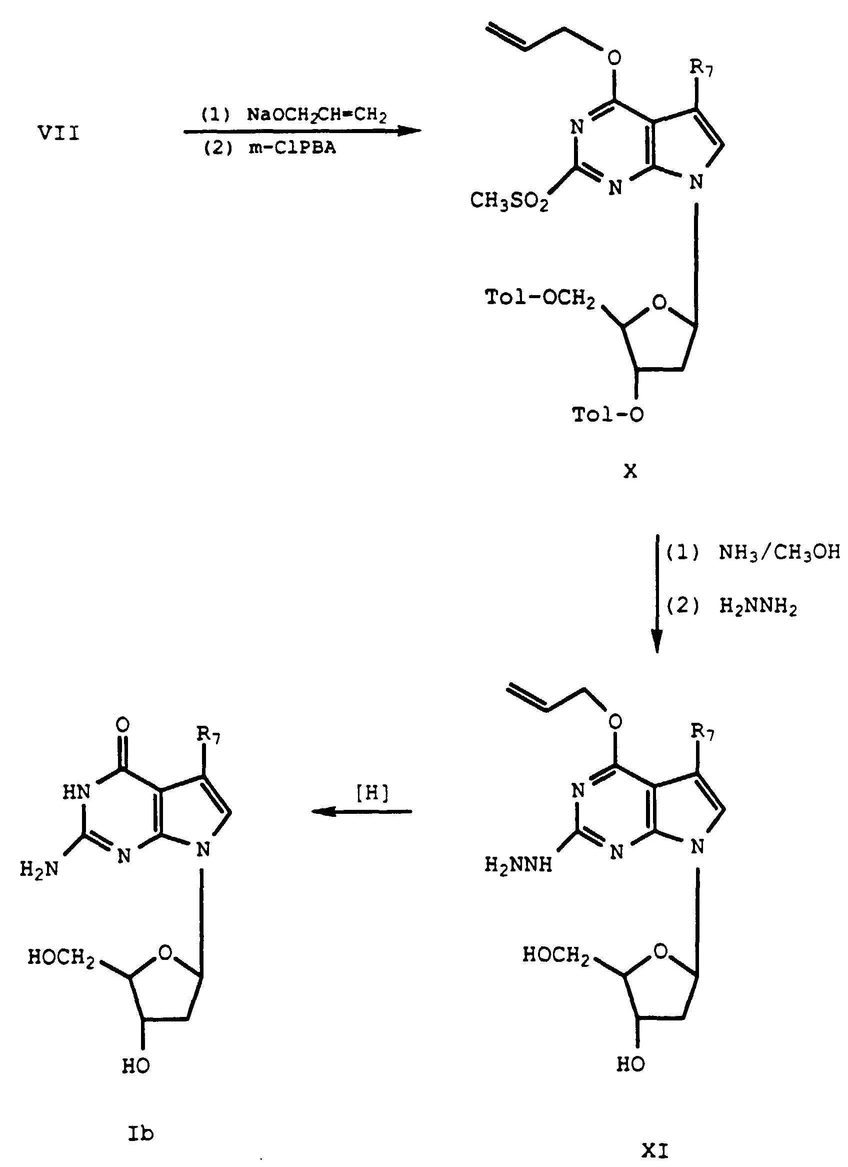

These can then be converted to the 9-β-D-2'-deoxyribofuranosyl-7-deaza-7-lower-alkylguanines of formula II, as described before.

The pharmaceutical compositions of the present invention include one or more of the compounds of this invention formulated into compositions together with one or more non-toxic physiologically acceptable carriers, adjuvants or vehicles which are collectively referred to herein as carriers, for parenteral injection, for oral administration in solid or liquid form, for rectal or topical administration, and the like.

The compositions can be administered to humans and animals either orally, rectally, parenterally (intravenously, intramuscularly or subcutaneously), intracisternally, intravaginally, intraperitoneally, locally (powders, ointments or drops), or as a buccal or nasal spray.

Compositions suitable for parenteral injection may comprise physiologically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminum monostearate and gelatin.

If desired, and for more effective distribution, the compounds can be incorporated into slow release or targeted delivery systems such as polymer matrices, liposomes, and microspheres. They may be sterilized, for example, by filtration through a bacteria-retaining filter, or by incorporating sterilizing agents in the form of sterile solid compositions which can be dissolved in sterile water, or some other sterile injectable medium immediately before use.

Solid dosage forms for oral administration include capsules, tablets, pills, powders and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol and silicic acid, (b) binders, as for example, carboxymethylcellulose, alginates, gelatin, polyvinylpyrrolidone, sucrose and acacia, (c) humectants, as for example, glycerol, (d) disintegrating agents, as for example, agar-agar, calcium carbonate, potato or tapioca starch alginic acid, certain complex silicates and sodium carbonate, (e) solution retarders, as for example, paraffin, (f) absorption accelerators, as for example, quaternary ammonium compounds, (g) wetting agents, as for example, cetyl alcohol and glycerol monostearate, (h) adsorbents, as for example, kaolin and bentonite, and (i) lubricants, as for example, talc, calcium stearate, magnesium stearate, solid polyethylene glycols, sodium lauryl sulfate or mixtures thereof. In the case of capsules, tablets and pills, the dosage forms may also comprise buffering agents.

The invention further relates to a method of inhibiting in vitro nuclease degradation of an oligonucleotide which comprises incorporating within said oligonucleotide modified 7-deaza-7-lower-alkyladenine nucleosides of the formula la or 7-deaza-7-lower-alkylguanine nucleosides of the formula Ib as defined in claim 1,
- either within the three nucleotide units at both the 3'-and the 5'- ends of the oligonucleotide sequence;
- or internally in the oligonucleotide sequence.

It should be understood that according to one of its aspects the invention also pertains to a composition for inhibiting gene expression comprising a modified oligonucleotide according to the invention in a pharmaceutically acceptable carrier.

The molecular structures of the compounds were established on the basis of a study of the nmr, infrared and mass spectra, and their purities were established by HPLC and chemical analysis for their elements.

### Nuclease Stability

Antisense oligonucleotides modified in accordance with the present invention were evaluated (and compared against unmodified oligonucleotides) for their stability in the presence of 10% (v/v) fetal bovine serum (FBS) in RPMI 1640 cell culture media containing 20 mM HEPES buffer (complete media). FBS and human serum are known to exhibit 3'→5' exonuclease activity. This represents the only nuclease activity we have been able to detect in FBS, human serum and human plasma. Oligonucleotide samples were incubated at 37°C in complete media over a six hour time period and the amount of parent compound determined using an HPLC-based procedure.

### Measurement of DNA/DNA Duplex Melting Temperatures

Oligonucleotide concentrations were determined spectrophotometrically utilizing extinction coefficients at 260 nm calculated using the method and values presented by Warsaw, Cantor, and Tinoco [CRC Handbook of Biochemistry and Molecular Biology (G.D. Fasman, editor) 1:589 (1975)]. Equimolar concentrations of oligonucleotide and its complementary sequence were combined (in 0.1 mM EDTA, 10 mM sodium phosphate, 0.1 M NaCl, pH 7.0) heated to 80°C and allowed to cool slowly at room temperature. Samples were allowed to remain at room temperature for about 2.5 hours. Samples were then heated at a rate of 0.5°C/min (25°C-75°C) in a thermostatically controlled heat block and absorbance monitored at 260 nm using a Perkin Elmer Lambda 4C UV spectrophotometer. A₂₆₀ measurements were taken every 15 seconds. Data were transferred to a DEC VAX for data analysis using RS/1 data analysis software. Tm's were determined from a plot of dA260/dT vs. temperature. The Tm is that temperature at which dA260/dT is maximum.

### Inhibition of Rabbit Alpha Globin mRNA Translation

Cell free translation of rabbit globin mRNA (Bethesda Res. Labs, Gaithersburg, MD) +/- 6.5 units/5 µl E.coli RNase H (Boehringer Mannheim, Indianapolis, IN) with the addition of antisense oligonucleotides was carried out using rabbit reticulocyte lysate (Promega, Madison, WI) in a total volume of 50 µl. 25 uCi of 35S-methionine (New England Nuclear, Boston, MA) was added to each translation reaction. Translations were incubated at 30°C for 10 min., after which time samples were snap frozen on dry ice. Alpha and beta globin chains were separated using SDS-polyacrylamide gel electrophoresis. The 15 cm gels were prepared using electrophoresis buffer (0.1 M sodium phosphate, pH 7.2 containing 1.0 g of SDS/l) and contained 12.5% acrylamide and 0.6% bisacrylamide. Aliquots (1 µl) of translation reactions were diluted with 11 uL of loading buffer consisting of electrophoresis buffer, 1.1% 2-mercaptoethanol, 2.5% glycerol and bromphenol blue. Samples were denatured by heating to 100°C for 3 min. before loading onto gels. The gels were run for 18 hours at 30 mAMP. After electrophoresis, gels were stained with coomassie blue, dried and autoradiographed at -70°C for 16 hours.

Quantitation of the effects of alpha globin directed antisense oligonucleotides on the synthesis of alpha globin was done by scanning the autoradiographs using an Ultrascan XL laser densitometer (LKB/Bromma) linked to an AT&T PC6300 computer. Data were collected, displayed and integrated with the Gelscan XL data analysis software package (LKB/Bromma). Effects of oligomers on protein synthesis were expressed as a percent of control alpha globin synthesis.

The following examples will further illustrate the invention without limiting it thereto. It will be apparent to those skilled in the art that the embodiments disclosed may be readily modified by standard procedures to produce oligonucleotides of other lengths and with other sequences. Targets for synthesis will usually be chosen by substituting a 7-deaza-7-lower-alkyladenine or 7-deaza-7-lower-alkylguanine nucleoside of formulas Ia or Ib in the sequence which is to be protected from nuclease degradation or which is complementary to a sequence which is to be blocked.

### Preparation of the Protected Nucleosides

### Preparation 1

A stirred suspension of 0.8 g (20 mmole) of a 60% sodium hydride in hexane dispersion was decanted and taken to dryness, resuspended in 100 ml of dry acetonitrile and the suspension treated with 3.21 g (15 mmole) of 4-chloro-5-methyl-2-methylthiopyrrolo[2,3-d]pyrimidine [Kondo et al., Agric. Biol. Chem. 4(8), 1501-1507 (1977)]. The mixture was stirred under nitrogen at room temperature for one hour and then treated with 5.9 g (15 mmole) of 1-chloro-2-deoxy-3,5-di-O-(p-toluoyl)-α-D-erythropentofuranose added in portions. An additional 40 ml of acetonitrile was added, the mixture stirred at 50°C for about three and one half hours and then filtered and the solid washed with acetonitrile and dried to give 6.1 g (72%) of 4-chloro-5-methyl-2-methylthio-7-[α-D-erythropentofuranosyl]pyrrolo[2,3-d]pyrimidine, m.p. 163-163.5°C.

The latter (11.0 g, 19.4 mmole) was suspended in 225 ml of a solution of absolute methanol saturated with ammonia and the mixture heated in an autoclave for nineteen hours at 125°C with stirring. The reaction mixture was cooled in an ice/water bath, taken to dryness in vacuo, the residue triturated sequentially with diethyl ether, chloroform and acetone and the solid collected and dried to give 2.65 g (45%) of 4-amino-5-methyl-2-methylthio-7-[α-D-erythro-pentofuranosyl]pyrrolo[2,3-d]pyrimidine, m.p. 187-189°C.

The product (0.47 g, 1.5 mmole) suspended in 45 ml of n-propanol was treated with 3.1 g of wet Raney nickel and the mixture heated under reflux for seven and a half hours, then cooled and filtered. The filtrate was taken to dryness and the residue recrystallized from water to give 0.26 g (72%) of 7-deaza-2'-deoxy-7-methyladenosine, m.p. 213.5-214.5°C.

A suspension of 0.22 g (0.83 mmole) of the product in 8.5 ml of dry pyridine was cooled in an ice/methanol bath and then treated dropwise with 0.5 ml (approximately 5 equivalents) of trimethylchlorosilane over a few minutes. The mixture was treated with about 0.5 ml of benzoyl chloride, stirred under nitrogen at room temperature for about two hours, cooled in an ice bath again, treated with 1.65 ml of water and 1.7 ml of concentrated ammonium hydroxide, stirred under nitrogen at ambient temperature for about a half hour and then taken to dryness. The crude product was triturated with water followed by cyclohexane to give 0.4 g of 6-dibenzoyl-7-deaza-2'-deoxy-7-methyladenosine, 6.5 g (11.35 mmole) of which was hydrolyzed to the mono 6-benzoyl-7-deaza-2'-deoxy-7-methyladenosine by treatment with 200 ml of a 50% solution of 1N sodium hydroxide in ethanol and then acidifying with 2N hydrochloric acid. There was thus obtained 3.61 g (86%) of product, m.p. 172-175°C.

The latter (1.75 g, 4.75 mmole), in about 50 ml of dry pyridine, was treated with 1.86 g (5.23 mmole) of 4,4'-dimethoxytrityl chloride and the mixture stirred at ambient temperature under nitrogen for about four hours and 17 mL of methanol added then taken to dryness in vacuo. The product was purified by chromatography on silica gel, eluting the product with 3% methanol in chloroform. There was thus obtained 1.97 g (62%) of 6-benzoyl-7-deaza-2'-deoxy-7-methyl-5'-dimethoxytrityladenosine, m.p. 112-115°C.

A solution of 0.9 g (1.3 mmole) of the product in 7.5 ml of dry THF was treated with 1.0 ml (5.7 mmole) diisopropylamine and the solution treated dropwise with 1.0 ml (4.5 mmole) of chloro-β-cyanoethoxy-N,N-diisopropylaminophosphine over a period of about 40 minutes while stirring under nitrogen. The mixture was then stirred at ambient temperature under nitrogen for about 40 minutes and taken to dryness in vacuo to give the crude product which was purified by chromatography on silica gel, the product being eluted with helium saturated ethyl acetate. There was thus obtained 0.62 g (55%) of 6-benzoyl-7-deaza-2'-deoxy-7-methyl-3'-O-[(N,N-diisopropylamino)-β -cyanoethoxyphosphanyl]-5'-dimethoxytrityladenosine, m.p. 73-76°C.

### Preparation 2

Reaction of the 4-chloro-5-methyl-2-methylthio-7-(α-D-erythro-pentofuranosyl)pyrrolo[2,3-d]pyrimidine described in Preparation 1 above with sodium 2-propenyloxide in DMF affords 5-methyl-2-methylthio-4-(2-propenyloxy)-7-(α-D-erythro-pentofuranosyl)pyrrolo[2,3-d]pyrimidine, which, on oxidation with two molar equivalents of 3-chloroperbenzoic acid in methylene chloride, affords 5-methyl-2-methylsulfonyl-4-(2-propenyloxy)-7-(α-D-erythro-pentofuranosyl)pyrrolo[2,3-d]-pyrimidine. Reaction of the product with hydrazine affords 5-methyl-2-hydrazino-4-(2-propenyloxy)-7-(α-D-erythro-pentofuranosyl)pyrrolo[2,3-d]pyrimidine. Reduction of the product with for example Raney nickel affords 7-deaza-2'-deoxy-7-methylguanosine.

Proceeding in a manner similar to that described in Preparation 1 above, the latter is treated sequentially first with trimethylchlorosilane in the presence of pyridine, then with isobutyric anhydride and then with concentrated ammonium hydroxide to give 2-isobutyryl-7-deaza-2'-deoxy-7-methylguanosine, which, on reaction with one molar equivalent of trityl chloride in the presence of dry pyridine, affords 2-isobutyryl-7-deaza-2'-deoxy-7-methyl-5'-tritylguanosine. Reaction of the latter with one molar equivalent of chloro-β-cyanoethoxy-N,N-diisopropylaminophosphine affords 2-isobutyryl-7-deaza-2'-deoxy-7-methyl-3'-O-[(N,N-diisopropylamino)-β-cyanoethoxyphosphanyl]-5'-tritylguanosine.

### Preparation of the Oligomers

### Examples 1-8

The oligomers of Examples 1-8 below, as well as the control samples, were synthesized using standard procedures on an Applied Biosystems model 380B DNA synthesizer to provide the DNA oligomers described below. In all successful couplings a 10-fold excess of the appropriate protected nucleoside monomer was used. In the examples, the letters A, G, W, X, C and T have the following nucleic acid base meanings:
- A:: adenine
- G:: guanine
- W:: 7-deazaadenine
- X:: 7-methyl-7-deazaadenine
- C:: cytosine
- T:: thymine

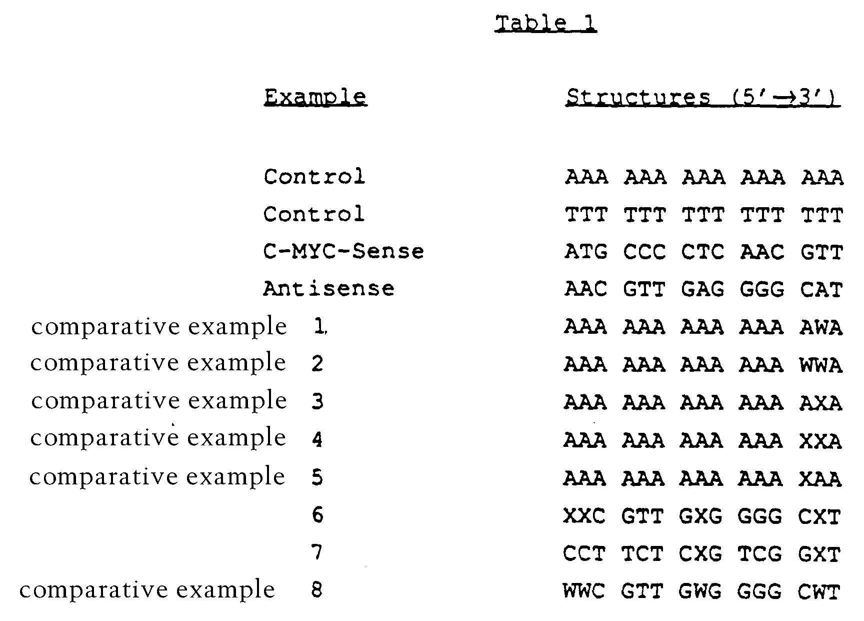

The melting temperatures obtained for each of the oligomers described above are given in Table 2 below.

**Table 2**

| Example | C (µm) | Tm (°C) | Δ Tm |
|---|---|---|---|
| Control (Antisense) | 7.5 | 42.2 | |
| Control (Antisense) | 8.8^{a} | 65.1^{a} | |
| comparative example 1 | 7.5 | 41.0 | -1.2 |
| comparative example 2 | 7.5 | 39.4 | -2.8 |
| comparative example 3 | 7.5 | 42.2 | 0.0 |
| comparative example 4 | 7.5 | 41.4 | -0.8 |
| comparative example 5 | 7.5 | 41.6 | -0.6 |
| 6 | | 64.5 | -0.6 |
| 7 | | | |
| comparative example 8 | 10^{a} | 63.4 | -1.7 |

| | | | |
|---|---|---|---|
| (a) Avg. of two values | | | |

The stabilities of the oligomers of the invention to 3-exonuclease, in comparison with controls, are given in Table 3 below.

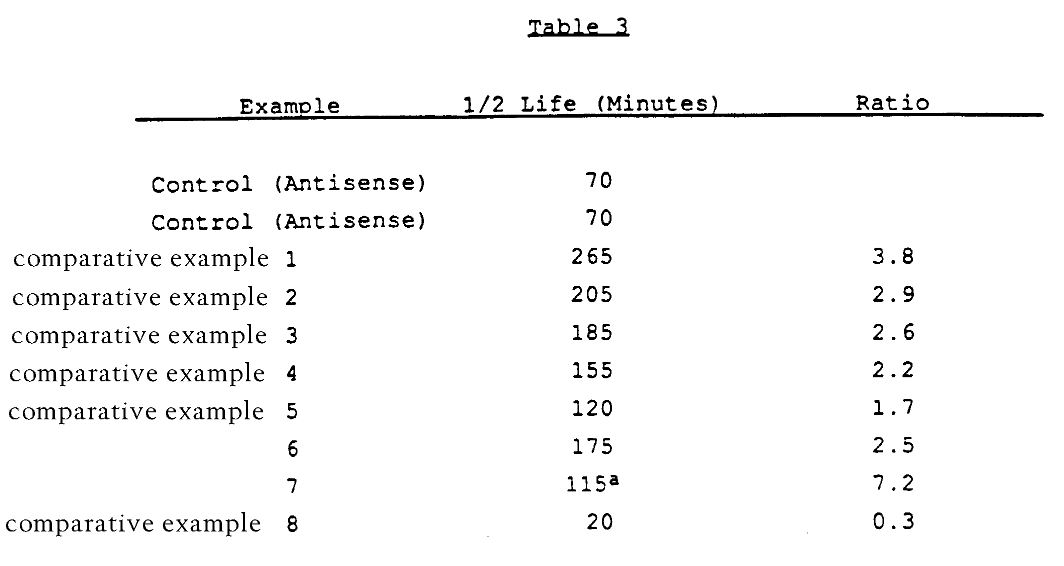

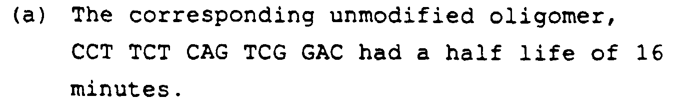

The oligomer of Example 6 was found to inhibit translation to 13 ± 4% of control in the absence of RNase H and 5 ± 1% in the presence of RNase H in comparison with corresponding unmodified oligomer which inhibited translation to 21 ± 4% of control in the absence of RNase H and 14% in the presence of RNase H.

## Claims

1. An oligonucleotide sequence of from 6 to 200 nucleotides containing one or more nucleotides incorporating the 7-deaza-7-lower-alkyladenine nucleosides of formula Ia or the 7-deaza-7-lower-alkylguanine nucleosides of formula Ib : 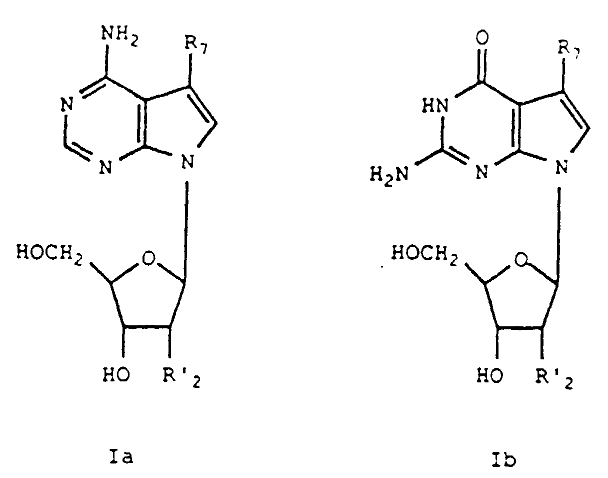 where :
R'₂ is hydrogen or hydroxy; and
R₇ is lower alkyl containing one to four carbon atoms
with the provisos that the oligonucleotides incorporate (1) no EcoRI endonuclease recognition site, (2) no repeating GC or CG sequences, and (3) no repeating AT or TA sequences,
said 7-deaza-7-lower-alkyladenine nucleosides of formula Ia and said 7-deaza-7-lower-alkyl-guanine nucleosides of formula Ib being :
- either incorporated within the three nucleotide units at both the 3'- and the 5'- ends of the oligonucleotide sequence;
- or incorporated internally in the oligonucleotide sequence.

2. An oligonucleotide according to Claim 1 wherein in the nucleotides derived from the nucleosides of formula Ia:
R'₂ is hydrogen and R₇ is lower-alkyl; or
R'₂ is hydroxy and R₇ is lower-alkyl and wherein in the nucleotides derived from the nucleosides of formula Ib:
R'₂ is hydroxy and R₇ is lower-alkyl; or
R'₂ is hydrogen and R₇ is lower-alkyl.

3. An oligonucleotide according to Claim 2 wherein R'₂ is hydrogen.

4. An oligonucleotide according to Claim 2 wherein R'₂ is hydroxy.

5. An oligonucleotide according to Claim 3 containing from 12 to 24 bases.

6. An oligonucleotide according to Claim 5 containing 15 bases.

7. An oligonucleotide according to Claim 4 containing from 12 to 24 bases.

8. An oligonucleotide according to Claim 7 containing 15 bases.

9. An oligonucleotide according to claim 1, selected from the group consisting of : XXC GTT GXG GGG CXT wherein;
G is guanine;
X is 7-methyl-7-deazaadenine;
C is cytosine; and
T is thymine.

10. A method of inhibiting in vitro nuclease degradation of an oligonucleotide which comprises incorporating within said oligonucleotide modified 7-deaza-7-lower-alkyladenine nucleosides of the formula Ia or 7-deaza-7-lower-alkylguanine nucleosides of the formula Ib as defined in claim 1,
- either within the three nucleotide units at both the 3'- and the 5'- ends of the oligonucleotide sequence;
- or internally in the oligonucleotide sequence.

11. Use of an oligonucleotide according to claim 1 for the preparation of a medicine intended to inhibit gene expression in a cellular system when introduced to the cellular system.

12. A composition for inhibiting gene expression comprising an oligonucleotide according to claim 1 in a pharmaceutically acceptable carrier.

## Patentansprüche

1. Oligonucleotidsequenz mit 6 bis 200 Nucleotiden, enthaltend ein oder mehrere Nucleotide, welche die 7-Deaza-7-niederalkyladeninnucleoside der Formel Ia oder die 7-Deaza-7-niederalkylguaninnucleoside der Formel Ib einschließen: 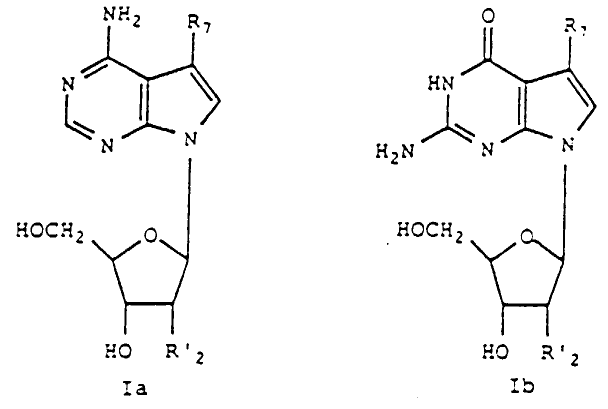 worin:
R'₂ Wasserstoff oder Hydroxy ist; und
R₇ Niederalkyl mit einem bis vier Kohlenstoffatomen ist,
mit den Maßgaben, daß die Oligonucleotide (1) keine EcoRI-Endonuclease-Erkennungsstelle, (2) keine wiederkehrenden GC- oder CG-Sequenzen und (3) keine wiederkehrenden AT- oder TA-Sequenzen einschließen.
wobei die 7-Diaza-7-niederalkyladeninnucleoside der Formel Ia und die 7-Deaza-7-niederalkylguaninnucleoside der Formel Ib
- entweder innerhalb der drei Nucleotideinheiten sowohl am 3'- als auch 5'-Ende der Oligonucleotidsequenz eingeschlossen sind;
- oder intern in der Oligonucleotidsequenz eingeschlossen sind.

2. Oliogonucleotid nach Anspruch 1, wobei in den von den Nucleosiden der Formel Ia abgeleiteten Nucleotiden
R'₂ Wasserstoff ist und R₇ Niederalkyl ist; oder
R'₂ Hydroxy ist und R₇ Niederalkyl ist.
und wobei in den von den Nucleosiden der Formel Ib abgeleiteten Nucleotiden
R'₂ Hydroxy ist und R₇ Niederalkyl ist; oder
R'₂ Wasserstoff ist und R₇ Niederalkyl ist.

3. Oligonucleotid nach Anspruch 2, wobei R'₂ Wasserstoff ist.

4. Oligonucleotid nach Anspruch 2, wobei R'₂ Hydroxy ist.

5. Oligonucleotid nach Anspruch 3, enthaltend 12 bis 24 Basen.

6. Oligonucleotid nach Anspruch 5, enthaltend 15 Basen.

7. Oligonucleotid nach Anspruch 4, enthaltend 12 bis 24 Basen.

8. Oligonucleotid nach Anspruch 7, enthaltend 15 Basen.

9. Oligonucleotid nach Anspruch 1, gewählt aus der XXC GTT GXG GGG CXT umfassenden Gruppe, worin
G Guanin ist;
X 7-Methyl-7-deazaadenin ist;
C Cytosin ist; und
T Thymin ist.

10. Verfahren zur Inhibierung des in-vitro-Nucleaseabbaus eines Oligonucleotids, umfassend das Einbringen innerhalb des Oligonucleotids von modifizierten 7-Deaza-7-niederalkyladeninnucleosiden der Formel Ia oder 7-Deaza-7-niederalkylguaninnucleosiden der Formel Ib wie in Anspruch 1 definiert.
- entweder innerhalb der drei Nucleotideinheiten sowohl am 3'- als auch 5'-Ende der Oligonucleotidsequenz;
- oder intern in die Oligonucleotidsequenz.

11. Verwendung eines Oligonucleotids nach Anspruch 1 zur Herstellung eines Arzneimittels, welches dazu dient, innerhalb eines Zellsystems die Genexpression zu inhibieren, wenn es in das Zellsystem eingebracht wird.

12. Zusammensetzung zur Inhibierung der Genexpression, umfassend ein Oligonucleotid nach Anspruch 1 in einem pharmazeutisch annehmbaren Träger.

## Revendications

1. Séquence oligonucléotidique de 6 à 200 nucléotides, contenant un ou plusieurs nucléotides incorporant les nucléosides à 7-déaza-7-(alkyl inférieur)adénine de formule Ia ou les nucléosides à 7-déaza-7-(alkyl inférieur)guanine de formule Ib: 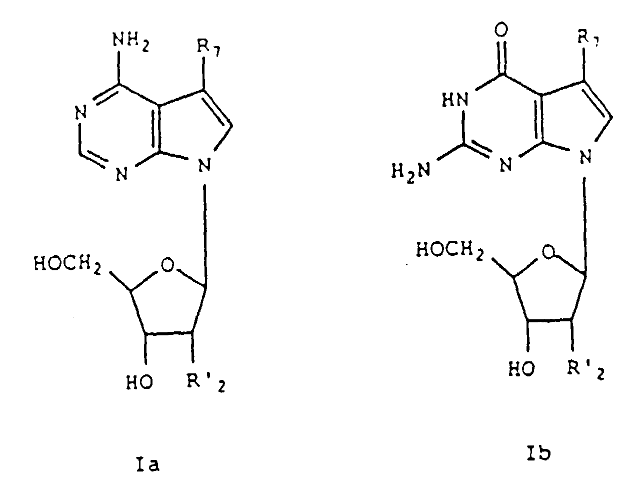 dans lesquelles :
R'₂ est un atome d'hydrogène ou le groupe hydroxy, et
R₇ est un groupe alkyle inférieur contenant de 1 à 4 atomes de carbone,
étant entendu que les oligonucléotides ne comprennent pas (1) de site de reconnaissance par l'endonucléase *Eco*RI, (2) de séquences répétitives GC ou CG, ni (3) de séquences répétitives AT ou TA,
ledits nucléosides à 7-déaza-7-(alkyl inférieur)adénine de formule Ia et lesdits nucléosides à 7-déaza-7-(alkyl inférieur)guanine de formule Ib étant:
- soit incorporés dans les limites des trois unités nucléotidiques à la fois à l'extrémité 3' et à l'extrémité 5' de la séquence oligonucléotidique;
- soit incorporés à l'intérieur de la séquence oligonucléotidique.

2. Oligonucléotide selon la revendication 1, dans lequel, dans les nucléotides dérivés des nucléosides de formule Ia:
R'₂ est un atome d'hydrogène et R₇ est un groupe alkyle inférieur, ou
R'₂ est le groupe hydroxy et R₇ est un groupe alkyle inférieur,
et dans lequel, dans les nucléotides dérivés des nucléosides de formule Ib:
R'₂ est le groupe hydroxy et R₇ est un groupe alkyle inférieur, ou
R'₂ est un atome d'hydrogène et R₇ est un groupe alkyle inférieur.

3. Oligonucléotide selon la revendication 2, dans lequel R'₂ est un atome d'hydrogène.

4. Oligonucléotide selon la revendication 2, dans lequel R'₂ est le groupe hydroxy.

5. Oligonucléotide selon la revendication 3, contenant de 12 à 24 bases.

6. Oligonucléotide selon la revendication 5, contenant 15 bases.

7. Oligonucléotide selon la revendication 4, contenant de 12 à 24 bases.

8. Oligonucléotide selon la revendication 7, contenant 15 bases.

9. Oligonucléotide selon la revendication 1, choisi dans le groupe consistant en: XXC GTT GXG GGG CXT dans lequel:
G est la guanine,
X est la 7-méthyl-7-déazaadénine,
C est la cytosine et
D est la thymine.

10. Procédé d'inhibition de la dégradation d'un oligonucléotide in vitro par des nucléases, comprenant l'incorporation dans ledit oligonucléotide de nucléosides à modification 7-déaza-7-(alkyl inférieur)adénine de formule Ia ou de nucléosides à modification 7-déaza-7-(alkyl inférieur)guanine de formule Ib, tels que définis dans la revendication 1,
- soit dans les limites des trois unités nucléotidiques à la fois à l'extrémité 3' et à l'extrémité 5' de la séquence oligonucléotidique;
- soit à l'intérieur de la séquence oligonucléotidique.

11. Utilisation d'un oligonucléotide selon la revendication 1, pour la fabrication d'un médicament destiné à inhiber l'expression de gènes dans un système cellulaire, lorsqu'il est introduit dans le système cellulaire.

12. Composition pour l'inhibition de l'expression de gènes, comprenant un oligonucléotide selon la revendication 1, dans un véhicule pharmaceutiquement acceptable.
